# EUROPEAN PATENT SPECIFICATION

(11) **EP 0 402 975 B1**
(45) Date of publication and mention of the grant of the patent: **10.08.1994**
(21) Application number: 90201363.0
(22) Date of filing: 29.05.1990
(51) Int. Cl.: C07C 69/753, C07C 219/12, C08G 61/00

(54) **Benzocyclobutene carboxylate esters**
Benzocyclobuten-Carboxylat-Ester
Esters benzocyclobutène carboxylates

(30) Priority: 12.06.1989 US 364275; 12.06.1989 US 364276; 23.06.1989 US 370507
(43) Date of publication of application: 19.12.1990
(73) Proprietor: SHELL INTERNATIONALE RESEARCH MAATSCHAPPIJ B.V., 2596 HR Den Haag (NL)
(72) Inventor: Wang, Pen-Chung, Houston, Texas 77079 (US)

(56) References cited:
- EP-A- 0 251 357
- US-A- 4 540 763
- CHEMICAL ABSTRACTS, vol. 69, no. 13, September 23, 1968, Columbus, Ohio, USA; I.L. KLUNDT et al, "Benzocyclobutenes. I. Nitration of 1-cyanobenzo-cyclobutene", page 4836, abstract-no. 51 859n

## Description

The curing of monomeric materials to produce thermoset resins is well known in the art. In general, the polymerizable monomers have at least one and customarily more than one active group which serves as the reactive site for a curing or crosslinking polymerization to produce the thermoset resins which are typically highly crosslinked. The curing or crosslinking of many if not most thermoset resins, for example, the curing of epoxy resins, requires the use of a curing agent, whether catalytic or stoichiometric, to cause the curing or crosslinking reaction to occur at an acceptable rate. Certain other monomers cure in the absence of added curing agent but only upon application of high intensity energy, e.g., ultraviolet (UV) light. Even in the presence of most curing agents the rate of crosslinking is often unduly slow and the addition of an accelerator is generally required to obtain sufficiently rapid curing.

There are some monomers in which the active sites are such that no added curing agent is required and such monomers cure upon application of heat. Such monomers are termed "self-curing". One class of such monomers includes within the molecular structure one or more moieties of a benzocyclobutene derivative. Such monomers are suitably cured by reaction with a conventional curing agent but also self-cure upon heating in the absence of a curing agent. Without wishing to be bound by any particular theory, it appears probably that upon application of heat the cyclobutene ring undergoes ring opening to produce active intermediates which crosslink by undergoing reaction with adjacent molecules. The resulting cured thermoset resins have properties of rigidity and strength. A series of U.S. patents to Kirchhoff, illustrated by U.S. 4,540,763, describes the production and curing of a large number of benzocyclobutene derivatives, including ethers of bis(hydroxyphenyl)alkanes, wherein the linking group connecting the phenyl of the bis(hydroxyphenyl)alkane to the benzocyclobutene moiety is attached directly to the six-membered ring of the benzocyclobutene. Such monomers are said to be self-curing, glass transition temperature of the cured resin is fairly modest.

The present invention claims as novel compounds 1-benzocyclobutenecarboxylate esters of a diglycidyl ether of a dihydric phenol or a N,N,N′,N′-tetraglycidyl-diamine.

1-Arylcyclobutenecarboxylic acids are known compounds. A to general review of arylcyclobutene chemistry, particularly benzocyclobutene chemistry, is provided by Klundt, Chemical Reviews Vol. 70, No. 4. pp. 471-487 (1970) and by the references provided in the review article. By way of a specific preparative example, 1-benzocyclobutenecarboxylic acid is produced by rearrangement of an α-diazaindanone.

The diglycidyl ethers of dihydric phenols represent a kn class of epoxy resins. The preferred phenol employed therein is 2,2-bis-(4-hydroxyphenyl)propane.

The N,N,N′,N′-tetraglycidyl-diamines are likewise known compounds. Preferred compounds are aromatic. The most preferred compounds comprise three benzene nuclei. The best results are obtained with 1,4-bis[2-(4-diglycidylaminophenyl)isopropyl]benzene.

To produce the novel benzocyclobutene derivatives of the invention, the 1-benzocyclobutenecarboxylic acid and the glycidyl ether compound or glycidyl amine compound are preferably employed in substantially equivalent quantities, that is, about 1 mole of benzocyclobutenecarboxylic acid for each glycidyl group present in the other compound.

The reaction is conducted in the substantial absence of reaction diluent when the reactants are liquid and reaction conditions or in the presence of an inert reaction diluent such as toluene in the case where one or both of the reactants is solid at reaction temperature and pressure. A satisfactory reaction rate is most easily obtained if the reaction of the 1-benzocyclobutenecarboxylic acid and the glycidyl compound are contacted in the presence of a catalyst. Quaternary phosphonium halides or quaternary ammonium halides have been found to be satisfactory as catalyst, particularly tetra(hydrocarbyl)phosphonium or tetra(hydrocarbyl)ammonium halides wherein at least one of the hydrocarbyl substituents is phenyl or alkyl with any other substituents being alkyl, particularly lower alkyl, and the halide is a middle halide, i.e., chloride or bromide. Illustrative of such phosphonium or ammonium halides are trimethylphenylphosphonium chloride, ethyltriphenylphosphonium bromide, tetrabutylammonium bromide, di-n-butyldiphenylphosphonium bromide and tetraphenylphosphonium chloride. Alkyltriphenylphosphonioum halides are preferred, especially ethyltriphenylphosphonium bromide. The phosphonium halide is employed in a catalytic quantity. Amounts of phosphonium halide from 1% by weight to 10% by weight, based on total reactants, are satisfactory with amounts from 2% by weight to 6% by weight on the same basis being preferred.

The reaction is conducted by intimately contacting the benzocyclobutenecarboxylic acid reactant, the glycidyl compound reactant, the catalyst and any diluent to be employed and maintaining the mixture under reaction conditions. An elevated reaction temperature is generally utilized and reaction temperatures from 30 °C to 200 °C are satisfactory with reaction temperatures from 50 °C to 150 °C being preferred. The reaction pressure to be employed is a pressure which is sufficient to maintain the reaction mixture in a liquid phase. Such pressures are typically up to 10 bar but more often are from 0.8 bar to 5 bar.

Subsequent to reaction, the desired benzocyclobutenecarboxylate ester is isolated from the product mixture. If desired, the ester product is separated and purified by conventional methods such as selective extraction, precipitation or solvent removal. Particularly in the embodiment of the invention where the reactions are employed in substantially stoichiometric ratios without the utilization of added diluent the product is obtained in sufficiently high conversion and selectivity so as to allow its use in most applications without the need for purification.

The benzocyclobutenecarboxylate esters of the bis or tetra (glycidyl) compounds are generally low melting solids or viscous liquids. Although the esters will react easily with curing agents. the esters are self-curing and will cure or crosslink without the presence of added curing agent or accelerator by heating the ester to a temperature above 100 °C, preferably from 150 °C to 250 °C. The cured products are rigid thermosets with a highly crosslinked structure and good physical strength. The esters are processed by methods which are conventional for curing monomeric compounds by application of heat. The cured products find utility as structural coating materials in aerospace and electronic applications. Whilst the novel compounds of this invention are self-curing, they may also be cured by reacting with a polymerizable monomer having at least two substituents selected from allyl, propargyl, styrylmethyl and non-hydrocarbon substituents, such as cyanato and maleimido, linked by a group selected from isocyanurate or 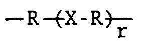
wherein R is phenylene, X is a direct valency bond, methylene, ethylene, propylene or butylene, or oxo or sulfonyl and r is 0 or 1, with the proviso that when the substituent linking group is isocyanurate, the substituents are allyl.

Illustrative of such additional polymerizable monomers are 1,9-decadiene, diallyl ether, di(3-cyanatophenyl)methane, 1,4-di(maleimido)biphenyl, 1,3-dipropargylbenzene, bis(4-maleimidophenyl)sulfone, 1-allyl-4-styrylmethylbenzene, 2,3-di(4-cyanatophenyl)propane, di(4-maleimidophenyl)methane and divinylbenzene. In the embodiment where the linking group is isocyanurate and the substituent group is allyl, the additional polymerizable monomer is triallylisocyanurate.

The compositions of the invention may comprise the 1-benzocyclobutenecarboxylate ester and at least one additional polymerizable monomer in an amount of from 1% by weight to 99% by weight based on total composition. In preferred embodiments of the invention, the 1-benzocyclobutenecarboxylate ester is present in a quantity of at least 40% by weight on the same basis with the additional polymerizable monomer(s) being present in a total of no more than 60% by weight. In more preferred embodiments, the 1-benzocyclobenzenecarboxylate ester is present in a quantity of at least 50% by weight.

The compositions of the invention are cured or crosslinked by application of heat. Curing or crosslinking is usually conducted by heating the composition to a curing temperature of at least 150 °C and preferably to a temperature from 175 °C to 300 °C. It is often desirable to effect the curing by heating in two states. Initially the composition is maintained at a relatively low curing temperature, e.g., from 175 °C to 210 °C, for a time sufficient to initiate crosslinking and then maintained at a higher curing, temperature to complete the cure. The cured products are high crosslinked solids having good properties of rigidity and strength. The compositions are processed by methods which are conventional for thermosetting resin compositions and are useful in coating and structural applications in the aerospace and electronic industries.

### EXAMPLE 1

A mixture of 29.6 g (0.2 mole) 1-benzocyclobutenecarboxylic acid, 34.0 g (0.1 mole) of diglycidyl ether of 2.2-bis(4-hydroxyphenyl)propane and 0.3 g of ethyltriphenylphosphonium bromide was stirred at 50 °C for 2 hours and at 100 °C for an additional 12 hours. The identity of the product as one of the novel compounds of the invention was confirmed by C13-NMR analysis which was consistent with the above structure. Yield of carboxylate ester was 99%. When heating this ester at 200 °C for 2 hours and at 220 °C for an additional 4 hours, a self-crosslinked, insoluble material having a glass transition temperature of 121 °C was obtained (self-curing).

### EXAMPLE 2

A mixture of 6.56 g (0.01 mole) of 1,4-bis[2-(4-diglycidylaminophenyl)isopropyl)benzene, 5.92 g (0.04 mole) of 1-benzocyclobutenecarboxylic acid and 0.2 g of ethyltriphenylphosphonium bromide was warmed to 100 °C-105 °C and maintained at that temperature until no further reaction took place. The resulting product was obtained in a yield greater than 99%. The C13-NMR spectra were consistent with the structure for N,N,N′,N′-tetra[3-(1-benzocyclobutenecarboxy)-2-hydroxy-1-propyl]-1,4-bis[2-(4-aminophenylisopropyl]benzene. When heating this product at 200 °C for two hours and at 220 °C for an additional two hours, a self-crosslinked, insoluble solid having a glass transition temperature of 125 °C was obtained.

### EXAMPLE 3

A mixture of equal parts by weight of the ester product of Example 1 and bis(4-maleimidophenyl)methane was melted at 100-120 °C. The resulting mixture was heated in an oven at 200 °C for 2 hours and then at 220 °C for an additional 4 hours. The resulting cured product had a glass transition temperature of 191 °C.

### EXAMPLE 4

A mixture of equal parts by weight of the ester of Example 1 and 2,2-bis(4-cyanatophenyl)propane was melted at 100-120 °C. The resulting mixture was heated in an oven at 200 °C for 2 hours and then at 220 °C for an additional 4 hours. The resulting cured material had a glass transition temperature of 112 °C.

### EXAMPLE 5

A mixture of equal parts by weight of the ester of Example 1 and triallylisocyanurate was melted at 100-120 °C. The resulting mixture was heated in an oven at 200 °C for 2 hours and then at 220 °C for an additional 4 hours. The resulting cured product had a glass transition temperature of 121 °C.

### EXAMPLE 6

A mixture of 50 parts by weight of the ester of Example 1, 5 parts by weight of bis(4-maleimidophenyl)methane and 45 parts by weight of 2,2-bis(4-cyanatophenyl)propane was heated at 100-120 °C. The resulting mixture was heated in an oven at 200 °C for 2 hours and then at 220 °C for an additional 4 hours. The resulting cured product had a glass transition temperature of 154°C.

## Claims (Claims for the following Contracting State(s): BE, DE, FR, GB, IT, NL)

1. A 1-benzocyclobutenecarboxylate ester of a diglycidyl ether of a dihydric phenol or a N,N,N',N'-tetraglycidyl-diamine.

2. The ester of claim 1 wherein the dihydric phenol is 2,2-bis-(4-hydroxyphenyl)propane.

3. The ester of claim 1 wherein the diamine is a trinuclear aromatic diamine.

4. The ester of claim 3 wherein the diamine is 1,4-bis[2-(4-diglycidylaminophenyl)isopropyl]benzene.

5. A curable composition comprising an ester of claims 1 to 4 and a polymerizable monomer having at least two substituents selected from allyl, propargyl, styrylmethyl, cyanato or maleimido, connected by a linking group selected from isocyanurate or 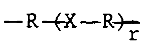 wherein R is phenylene, X is a direct valency bond, methylene or ethylene, propylene or butylene or oxo or sulfonyl and r is 0 or 1, with the proviso that when the linking group is isocyanurate the substituents are allyl.

6. The composition of claim 5 wherein the monomer is bis-(4-maleimidophenyl)methane, 2,2-bis-(4-cyanatophenyl)propane or triallylisocyanurate.

## Claims (Claims for the following Contracting State(s): ES)

1. A process for the preparation of a 1-benzocyclobutenecarboxylate ester by reacting a benzocyclobutenecarboxylic acid with a diglycidyl ether of a dihydric phenol or a N,N,N',N'-tetraglycidyl-diamine in substantially equivalent quantities.

2. A process as claimed in claim 1, wherein the dihydric phenol is 2,2-bis-(4-hydroxyphenyl)propane.

3. A process as claimed in claim 1, wherein the diamine is a trinuclear aromatic diamine.

4. A process as claimed in claim 3, wherein the diamine is 1,4-bis[2-(4-diglycidylaminophenyl)isopropyl]benzene.

5. A process for the preparation of a curable composition by blending an ester prepared according to a process as claimed in claims 1 to 4 with a polymerizable monomer having at least two substituents selected from allyl, propargyl, styrylmethyl, cyanato or maleimido, connected by a linking group selected from isocyanurate or 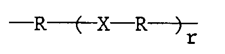 wherein R is phenylene, X is a direct valency bond, methylene or ethylene, propylene or butylene or oxo or sulfonyl and r is 0 or 1, with the proviso that when the linking group is isocyanurate the substituents are allyl.

6. A process as claimed in claim 5, wherein the monomer used is bis-(4-maleimidophenyl)methane, 2,2-bis-(4-cyanatophenyl)propane or triallylisocyanurate.

## Patentansprüche (Patentansprüche für folgende(n) Vertragsstaat(en): BE, DE, FR, GB, IT, NL)

1. Ein 1-Benzocyclobuten-Carboxylat-Ester eines Diglycidylethers eines zweiwertigen Phenols oder eines N,N,N,N'-Tetraglycidyl-diamins.

2. Der Ester des Anspruchs 1, wobei das zweiwertige Phenol 2,2-Bis-(4-hydroxyphenyl)propan ist.

3. Der Ester des Anspruchs 1, wobei das Diamin ein dreikerniges aromatisches Diamin ist.

4. Der Ester des Anspruchs 3, wobei das Diamin 1,4-Bis-[2-(4-diglycidylaminophenyl)isopropyl]benzol ist.

5. Eine härtbare Zusammensetzung, umfassend einen Ester der Ansprüche 1 bis 4 und ein polymerisierbares Monomer mit wenigstens zwei Substituenten, ausgewählt aus Allyl, Propargyl, Styrylmethyl, Cyanato oder Maleimido, verbunden durch eine Bindungsgruppe, ausgewählt aus Isocyanurat oder 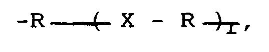 wobei R Phenylen ist, X eine direkte Valenzbindung, Methylen oder Ethylen, Propylen oder Butylen oder Oxo oder Sulfonyl ist und r Null oder 1 ist, mit der Maßgabe, daß, wenn die Bindungsgruppe Isocyanurat ist, die Substituenten Allyl sind.

6. Die Zusammensetzung des Anspruchs 5, wobei das Monomer Bis(4-maleimidophenyl)methan, 2,2-Bis(4-cyanatophenyl)propan oder Triallylisocyanurat ist.

## Patentansprüche (Patentansprüche für folgende(n) Vertragsstaat(en): ES)

1. Ein Verfahren zur Herstellung eines 1-Benzocyclobutan-Carboxylat-Esters durch Umsetzen einer Benzocyclobutencarbonsäure mit einem Diglycidylether eines zweiwertigen Phenols oder einem N,N,N',N'-Tetraglycidyl-diamin in im wesentlichen äquivalenten Mengen.

2. Ein Verfahren wie in Anspruch 1 beansprucht, wobei das zweiwertige Phenol 2,2-Bis-(4-hydroxyphenyl)propan ist.

3. Ein Verfahren wie in Anspruch 1 beansprucht, wobei das Diamin ein dreikerniges aromatisches Diamin ist.

4. Ein Verfahren wie in Anspruch 3 beansprucht, wobei das Diamin 1,4-Bis-[2-(4-diglycidylaminophenyl)isopropyl]benzol ist.

5. Ein Verfahren zur Herstellung einer härtbaren Zusammensetzung durch Vermischen eines nach einem Verfahren, wie in den Ansprüchen 1 bis 4 beansprucht, hergestellten Esters mit einem polymerisierbaren Monomeren mit wenigstens zwei Substituenten, ausgewählt aus
Allyl, Propargyl, Styrylmethyl, Cyanato oder Maleimido, verbunden durch eine Bindungsgruppe, ausgewählt aus Isocyanurat oder 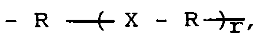 wobei R Phenylen ist, X eine direkte Valenzbindung, Methylen oder Ethylen, Propylen oder Butylen oder Oxo oder Sulfonyl ist und r Null oder 1 ist, mit der Maßgabe, daß, wenn die Bindungsgruppe Isocyanurat ist, die Substituenten Allyl sind.

6. Ein Verfahren wie in Anspruch 5 beansprucht, wobei das verwendete Monomer Bis-(4-maleimidophenyl)methan, 2,2-Bis-(4-cyanatophenyl)propan oder Triallylisocyanurat ist.

## Revendications (Revendications pour l'(les) Etat(s) contractant(s) suivant(s): BE, DE, FR, GB, IT, NL)

1. Ester 1-benzocyclobutènecarboxylate d'un éther diglycidylique d'un phénol dihydroxylé ou d'une N,N,N',N'-tètraglycidyl-diamine.

2. Ester selon la revendication 1, dans lequel le phénol dihydroxylé est le 2,2-bis-(4-hydroxyphényl)propane.

3. Ester selon la revendication 1, dans lequel la diamine est une diamine aromatique trinucléaire.

4. Ester selon la revendication 3, dans lequel la diamine est le 1,4-bis(2-(4-diglycidylaminophényl)isopropyl)benzène

5. Composition durcissable qui comprend un ester selon les revendications 1 à 4 et un monomère polymérisable possédant au moins deux substituants choisis parmi les groupes allyle, propargyle, styrylméthyle, cyanato ou maléimido, liés par un groupe de liaison choisi parmi l'isocyanurate ou 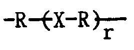 où R est un groupe phénylène, X est une liaison de valence directe, un groupe méthylène ou éthylène, propylène ou butylène ou oxo ou sulfonyle et r vaut 0 ou 1, à la condition que lorsque le groupe de liaison est l'isocyanurate, les substituants sont des groupes allyle.

6. Composition selon la revendication 5, dans laquelle le monomère est le bis(4-maléimidophényl)méthane, le 2,2-bis-(4-cyanatophényl)propane ou le triallylisocyanurate.

## Revendications (Revendications pour l'(les) Etat(s) contractant(s) suivant(s): ES)

1. Procédé de préparation d'un ester 1-benzocyclobutènecarboxylate par réaction d'un acide benzocyclobutènecarboxylique avec un éther diglycidylique d'un phénol dihydroxylé ou une N,N,N',N'-tétra-glycidyl-diamine dans des quantités pratiquement équivalentes.

2. Procédé selon la revendication 1, dans lequel le phénol dihydroxylé est le 2,2-bis-(4-hydroxyphényl)propane.

3. Procédé selon la revendication 1, dans lequel la diamine est une diamine aromatique trinucléaire.

4. Procédé selon la revendication 3, dans lequel la diamine est le 1,4-bis(2-(4-diglycidylaminophényl)isopropyl)benzène

5. Procédé de préparation d'une composition durcissable par mélange d'un ester préparé conformément au procédé tel que revendiqué dans les revendications 1 à 4, avec un monomère polymérisable possédant au moins deux substituants choisis parmi les groupes allyle, propargyle, styrylméthyle, cyanato ou maléimido, liés par un groupe de liaison choisi parmi l'isocyanurate ou 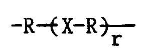 où R est un groupe phénylène, X est une liaison de valence directe, un groupe méthylène ou éthylène, propylène ou butylène ou oxo ou sulfonyle et r vaut 0 ou 1, à la condition que lorsque le groupe de liaison est l'isocyanurate, les substituants sont des groupes allyle.

6. Procédé selon la revendication 5, dans lequel le monomère utilisé est le bis(4-maléimidophényl)méthane, le 2,2-bis-(4-cyanatophényl)propane ou le triallylisocyanurate.
